# EUROPEAN PATENT SPECIFICATION

(11) **EP 3 638 172 B1**
(45) Date of publication and mention of the grant of the patent: **27.07.2022**
(21) Application number: 18735164.8
(22) Date of filing: 12.06.2018
(51) Int. Cl.: A61F 13/00, A61F 13/02

(54) **FOAMED AND TEXTURED SINTERED POLYMER WOUND FILLER**
GESCHÄUMTER UND TEXTURIERTER GESINTERTER POLYMERWUNDFÜLLER
CHARGE POUR PLAIE EN POLYMÈRE FRITTÉ TEXTURÉ ET EXPANSÉ

(30) Priority: 12.06.2017 US 201762518309 P
(43) Date of publication of application: 22.04.2020
(73) Proprietor: 3M Innovative Properties Company, St. Paul, MN 55133-3427 (US)
(72) Inventor: ROBINSON, Timothy Mark, Shillingstone DT11 0TG (GB); LOCKE, Christopher Brian, Bournemouth BH9 3SD (GB); ALLEN, Diwi L., San Antonio, TX 78255 (US); COURAGE, James, San Antonio, TX 78232 (US)
(74) Representative: Simmons & Simmons
(86) International application number: PCT/US2018/037099
(87) International publication number: WO 2018/231825

(56) References cited:
- EP-A1- 0 588 321
- WO-A1-2007/023089
- US-A1- 2008 195 017

## Description

### TECHNICAL FIELD

The invention set forth in the appended claims relates to making a foamed and textured sintered polymer wound filler for use in conjunction with reduced pressure wound therapy.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "reduced-pressure therapy," but is also known by other names, including "reduced-pressure wound therapy," "negative-pressure therapy," "negative pressure therapy," "vacuum therapy," "vacuum-assisted closure," and "topical negative-pressure," for example. Reduced-pressure therapy may provide a number of benefits, including migration of epithelial and subcutaneous tissues, improved blood flow, and micro-deformation of tissue at a wound site. Together, these benefits can increase development of granulation tissue and reduce healing times.

Tissue treatment systems can use a tissue interface (such as a wound manifold or a wound filler) that when installed at a tissue site (such as on a wound or in a wound at a tissue site) facilitates various degrees of tissue ingrowth that can cause pain or discomfort to a patient and in some cases leave remnants of the tissue interface in the wound when the tissue interface is removed from the tissue site. Accordingly, improvements to the tissue interface are desirable.

While the clinical benefits of reduced-pressure therapy and tissue interfaces are widely known, improvements to therapy systems, components, and processes may benefit healthcare providers and patients.

### BRIEF SUMMARY

New and useful methods for manufacturing tissue interfaces are set forth in the appended claims. Illustrative embodiments are also provided to enable a person skilled in the art to make and use the claimed subject matter.

A method of manufacturing a tissue interface for providing reduced pressure to a tissue site is provided. The method includes foaming a plurality of polymer particles to form a porous polymer. The method also includes sintering the porous polymer to form a sintered porous polymer. The method further includes texturing the sintered porous polymer to form a textured sintered polymer that is porous. In addition, the method includes sterilizing the tissue interface.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a simplified functional block diagram of an example embodiment of a therapy system for providing reduced pressure therapy comprising a tissue interface;
Figure 2 is a perspective view of a first example embodiment of a tissue interface for use in the therapy system of Figure 1;
Figure 3 is an exploded view of a portion of the first example embodiment of the tissue interface of Figure 2;
Figure 4 is a perspective view of a second example embodiment of a tissue interface for use in the therapy system of Figure 1; and
Figure 5 is an exploded view of a portion of the second example embodiment of the tissue interface of Figure 4.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

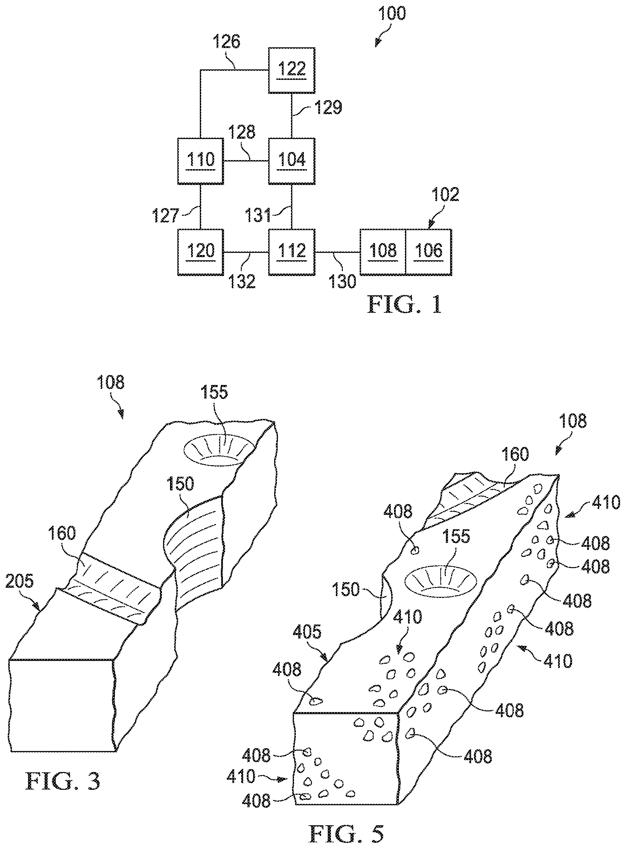

Figure 1 is a simplified functional block diagram of an example embodiment not according to the invention of a therapy system 100 for providing reduced-pressure therapy comprising a tissue interface including an embossed mesh film or an embossed, manifolding wound filler.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, reduced pressure may be applied to a tissue site to grow additional tissue that may be harvested and transplanted.

The therapy system 100 may include reduced-pressure supply, and may include or be configured to be coupled to a distribution component or tubing, such as a dressing. In general, a distribution component may refer to any complementary or ancillary component configured to be fluidly coupled to a reduced-pressure supply in a fluid path between a reduced-pressure supply and a tissue site. A distribution component is preferably detachable, and may be disposable, reusable, or recyclable. For example, a dressing 102 may be fluidly coupled to a reduced-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the reduced-pressure source 104.

A dressing interface may facilitate coupling the reduced-pressure source 104 to the dressing 102. For example, such a dressing interface may be a T.R.A.C.^{®} Pad or Sensa T.R.A.C.^{®} Pad available from KCI of San Antonio, Texas. The therapy system 100 may optionally include a fluid container, such as a container 112, coupled to the dressing 102 and to the reduced-pressure source 104.

Additionally, the therapy system 100 may include sensors to measure operating parameters and provide feedback signals to the controller 110 indicative of the operating parameters. As illustrated in Figure 1, for example, the therapy system 100 may include at least one of a pressure sensor 120 or an electric sensor 122 coupled to the controller 110 via electric conductors 126 and 127, respectively. The pressure sensor 120 may also be coupled or configured to be fluidly coupled via a distribution component such as, for example, fluid conduits 131 and 132 and to the reduced-pressure source 104. For example, as shown in Figure 1, the electric conductor 126 provides electric communication between the electric sensor 122 and the controller 110. The electric conductor 127 provides electric communication between the pressure sensor 120 and the controller 110. The electric conductor 128 provides electric communication between the controller 110 and the reduced-pressure source 104. The electric conductor 129 provides electric communication between the electric sensor 122 and the reduced-pressure source 104.

Components may be fluidly coupled to each other to provide a path for transferring fluids (such as at least one of liquid or gas) between the components. Components may be fluidly coupled through a fluid conductor, such as a tube. For example fluid conductor 130 provides fluid communication between the dressing 102 and the container 112; fluid conductor 131 provides fluid communication between the reduced-pressure source 104 and the container 112; and fluid conductor 132 provides fluid communication between the pressure sensor 120 and the container 112. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors 124 may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the reduced-pressure source 104 may be directly coupled to the controller 110, and may be indirectly coupled to the dressing 102 through the container 112.

The fluid mechanics of using a reduced-pressure source to reduce pressure in another component or location, such as within a sealed therapeutic environment, can be mathematically complex. However, the basic principles of fluid mechanics applicable to reduced-pressure therapy are generally well-known to those skilled in the art, and the process of reducing pressure may be described illustratively herein as "delivering," "distributing," or "generating" reduced pressure, for example.

In general, exudates and other fluids flow toward lower pressure along a fluid path. Thus, the term "downstream" typically implies something in a fluid path relatively closer to a source of reduced pressure or further away from a source of positive pressure. Conversely, the term "upstream" implies something relatively further away from a source of reduced pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a reduced-pressure source) and this descriptive convention should not be construed as a limiting convention.

"Reduced pressure" or "negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in reduced pressure typically refer to a decrease in absolute pressure, while decreases in reduced pressure typically refer to an increase in absolute pressure. While the amount and type of reduced pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A reduced-pressure supply, such as the reduced-pressure source 104, may be a reservoir of air at a reduced pressure, or may be a manual or electrically-powered device that can reduce the pressure in a sealed volume, such as a vacuum pump, a suction pump, a wall suction port available at many healthcare facilities, or a micro-pump, for example. A reduced-pressure supply may be housed within or used in conjunction with other components, such as sensors, processing units, alarm indicators, memory, databases, software, display devices, or user interfaces that further facilitate therapy. For example, in some embodiments, the reduced-pressure source 104 may be combined with the controller 110 and other components into a therapy unit. A reduced-pressure supply may also have one or more supply ports configured to facilitate coupling and de-coupling the reduced-pressure supply to one or more distribution components.

Sensors, such as the pressure sensor 120 or the electric sensor 122, are generally known in the art as any apparatus operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the reduced-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 may be representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with reduced-pressure therapy.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a reduced pressure at a tissue site for a given reduced-pressure source. The cover 106 may have a high moisture-vapor transmission rate (MVTR) in some applications. For example, the MVTR may be at least 300 g/m^2 per twenty-four hours in some embodiments. In some example embodiments, the cover 106 may be a polymer drape, such as a polyurethane film, that is permeable to water vapor but impermeable to liquid. Such drapes typically have a thickness in the range of 25-50 microns. For permeable materials, the permeability generally should be low enough that a desired reduced pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The tissue interface 108 may be configured to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the type and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive reduced pressure from a source and distribute reduced pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In operation, the tissue interface 108 may be placed within, over, on, or otherwise proximate to a tissue site. The cover 106 may be placed over the tissue interface 108 and sealed to an attachment surface near the tissue site. For example, the cover 106 may be sealed to undamaged epidermis peripheral to a tissue site. Thus, the dressing 102 can provide a sealed therapeutic environment proximate to a tissue site, substantially isolated from the external environment, and the reduced-pressure source 104 can reduce the pressure in the sealed therapeutic environment. Reduced pressure applied across the tissue site through the tissue interface 108 in the sealed therapeutic environment can induce macrostrain and microstrain in the tissue site, as well as remove exudates and other fluids from the tissue site, which can be collected in container 112.

The tissue interface 108 may comprise a sheet-like material or other-shaped material comprising a polymer. Polymers may include substances having a molecular structure consisting predominantly or entirely of similar units bonded together. In various example embodiments, the tissue interface 108 may include a polymer that may be porous or nonporous, perforated, textured, or sintered to enhance wound therapy. The tissue interface 108 may also include indentations or hollows in the surface of the sheet-like material. For example, the tissue interface 108 may comprise a porous polymer formed from a sheet of foam material or a sheet of compressed particles. In another example embodiment, the tissue interface 108 may include a porous polymer formed from a sheet of foam or compressed particles that is sintered or subjected to other processes. The tissue interface 108 may also comprise a nonporous polymer that may be formed from a sheet of nonporous material, and that may be perforated, textured, or subject to other processes as described in more detail below.

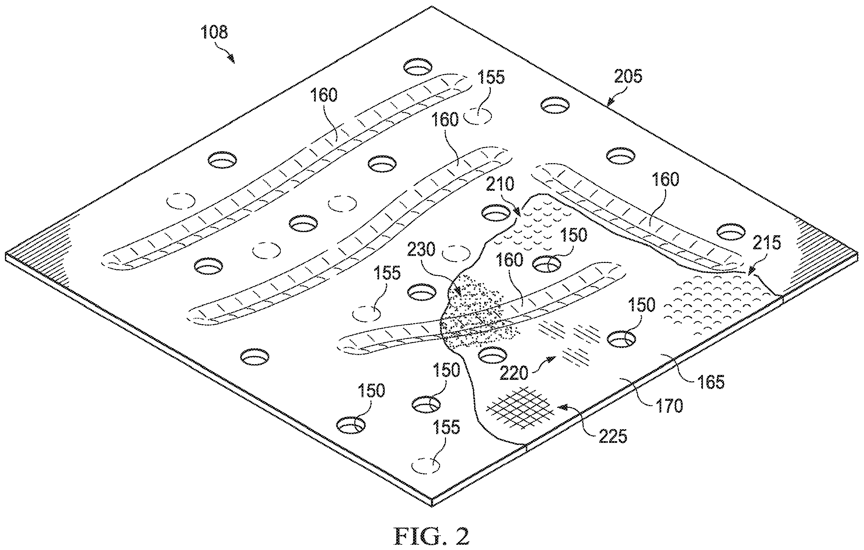

Referring more specifically to Figures 2 and 3, the tissue interface 108 comprises a sheet-like material that comprises a nonporous polymer 205 that is shown as being perforated and textured. The tissue interface 108 may be perforated as described above to include one or more fenestrations or perforations 150 extending through any of the embodiments of the tissue interface 108 described above. For example, the nonporous polymer 205 of the tissue interface 108 may comprise perforations 150. The perforations 150 may communicate fluid through the tissue interface 108 to enhance wound therapy including negative pressure wound therapy. The tissue interface 108 may also include a surface having one or more indentations or hollows on the surface that do not extend through the nonporous material 205. Such indentations or hollows may have a variety of shapes such as, for example, one or more dimples 155 or channels 160.

As indicated above, the tissue interface 108 may have a generally sheet-like shape which may include a textured surface 165 on at least one side of the sheet or a portion 170 of one side of the sheet. In one example embodiment, the textured surface 165 of the tissue interface 108 may include a pattern or arrangement of particles or constituent parts of the tissue interface 108. In yet another example embodiment, the textured surface 165 of the tissue interface 108 may include any flexible or rigid protrusions extending from one side of the sheet. Such patterns or protrusions may have a variety of shapes such as, for example, pyramids, cylinders, ribs, or other non-symmetrical shapes. The protrusions may be arranged in regular or irregular patterns, and the patterns themselves may be irregular and/or non-symmetrically formed on the surface of the tissue interface 108. The protrusions of the textured surface 165 may be formed with either a course or fine grain surface. The textured surface 165 may be formed by embossing methods that cover the surface of the tissue interface 108 with patterns of protrusions raised above the surface of the tissue interface 108. For example, as shown in Figure 2, the textured surface 165 may include dimples 210, protrusions or bumps 215, scuffs 220, a scored portion 225, a roughed or sand-blasted portion 230, or any other texture known by those having ordinary skill in the art.

In some embodiments of the tissue interface 108 having perforations 150, the perforations 150 may be formed by punching holes through the tissue interface 108, perforating the tissue interface 108, cutting holes in the tissue interface 108, or any number of different methods. The perforations 150 also may be formed by vacuum forming methods. The perforations 150 may be formed into one or more shapes including, for example, an elliptical shape including a circle, a polygon, an oval, or a simple slit through the material. Perforations 150 having a generally elliptical shape may have diameters between about 300 (µm) microns and about 1000 µm. The perforations 150 may be stretched in different directions to change the shape of the perforations 150. Stretching the tissue interface 108 in one or more directions to form different shapes and sizes facilitates fluid communication through the tissue interface 108. The perforations 150 may be positioned randomly throughout the tissue interface 108. In at least some embodiments, the perforations 150 may be positioned so that one or more perforations 150 are more concentrated in one region of the tissue interface 108 while other perforations 150 are less concentrated in other regions of the tissue interface 108. In some embodiments of the tissue interface 108, the perforations 150 may be formed through the tissue interface 108 in combination with one or more of the other features described herein. For example, the perforations 150 may be through-holes extending through the tissue interface 108 from the base of the dimples 155 or the base the channels 160 (not shown) rather than from the surface of the tissue interface 108.

As indicated herein, the tissue interface 108 may also include one or more dimples 155. The dimples 155 may be positioned or formed on a surface of the tissue interface 108. For example, when the tissue interface 108 includes a porous polymer, the dimples 155 may create more surface area on the surface of the tissue interface 108 to facilitate absorption of fluids into pores of the tissue interface 108. The dimples 155 may be formed on the surface of the tissue interface 108 by texturing the surface of the tissue interface 108. The tissue interface 108 may be textured to form the one or more dimples 155 by pressing a pattern of dimples 155 into the surface of the tissue interface 108, by vacuum forming a pattern of dimples 155 into the surface of the tissue interface 108, or by any other method known by those skilled in the art. At least one of the dimples 155 may have a diameter between about 50 µm and about 2000 µm. The dimples 155 may have a hollow shape including the shape of a cone, an ellipsoid, a hemisphere, or a polyhedron. The dimples 155 also may have a depth between about 200 µm and about 1000 µm. The dimples 155 may have a variety of different shapes and sizes positioned where formed on the surface of the tissue interface 108. The dimples 155 may be positioned or formed randomly on the tissue interface 108 in an irregular pattern. In at least some embodiments, the dimples 155 may be positioned so that some of the dimples 155 are more concentrated in at least one region of the tissue interface 108 while other dimples 155 are less concentrated in another region of the tissue interface 108.

As indicated above, the tissue interface 108 may also include one or more channels 160. The channels 160 may be positioned or formed on a surface of the tissue interface 108. For example, when the tissue interface 108 includes a porous polymer, the channels 160 may channel or guide fluid along a surface of the tissue interface 108. The channels 160 may be positioned or formed randomly on the tissue interface 108 in an irregular pattern. The channels 160 may be formed on the surface the tissue interface 108 by texturing the surface of the tissue interface 108. The tissue interface 108 may be textured to form the channels 160 by embossing the tissue interface 108 (for example using embossing rollers), pressing a pattern of channels 160 into the surface of the tissue interface 108, vacuum forming the tissue interface 108, or by any other method known by those skilled in the art. The channels 160 may have a variety of different shapes and sizes positioned where formed on the surface of the tissue interface 108. The channels 160 may have a linear shape, a curved shape, or an angular shape formed on a surface of the tissue interface 108. The channels 160 may have a cross-sectional shape including at least one of a semicircular shape, and popular shape, a V-shape, circle, or any other geometric shape known by those skilled in the art. At least one of the channels 160 may have a depth between about 200 µm and about 1000 µm and a width between about 200 µm and about 1000 µm. In various embodiments, at least one of the channels 160 may be positioned or formed on the surface of the tissue interface 108 to intersect with other features described herein including at least one of a perforation 150, a dimple 155, or another channel 160. In at least some embodiments, the channels 160 may be positioned so that some of the channels 160 are more concentrated in at least one region of the tissue interface 108 while other channels 160 are less concentrated in another region of the tissue interface 108.

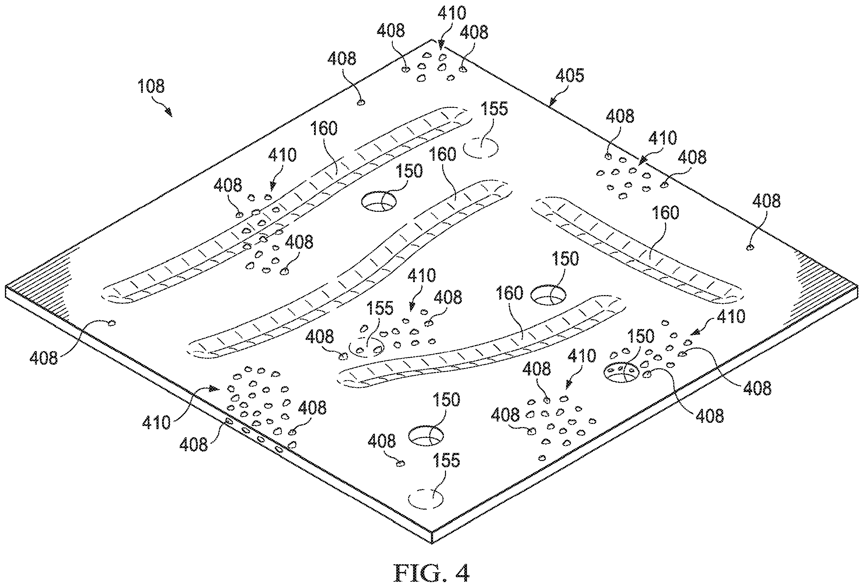

Referring more specifically to Figures 4 and 5, the tissue interface 108 comprises a sheet-like material that comprises a porous polymer 405 that is shown as being perforated and textured. The tissue interface 108 may be perforated as described above to include one or more fenestrations or perforations 150 extending through any of the embodiments of the tissue interface 108 described above. For example, the porous polymer 405 of the tissue interface 108 may comprise perforations 150. The perforations 150 may communicate fluid through the tissue interface 108 to enhance wound therapy including negative pressure wound therapy. The tissue interface 108 may also include a surface having one or more indentations or hollows on the surface that do not extend through the porous polymer 405. Such indentations or hollows may have a variety of shapes such as, for example, one or more dimples 155 or channels 160. The porous polymer 405 may comprise at least some of the features described above respect to the nonporous material 205 shown in Figures 2 and 4. For example, the tissue interface 108 may comprise porous polymer 405 formed from a sheet of foam material or a sheet of compressed particles that is textured or subject to other processes. The porous polymer 405 may include one or more voids or pores 408 that are positioned or formed throughout the tissue interface 108. For example, the porous polymer 405 may include pores 408 that are positioned or formed uniformly throughout the tissue interface 108. Alternatively, the pores 408 may be formed more randomly throughout the tissue interface 108. As another example, the porous polymer 405 may include pores 408 that are positioned or formed in clusters 410 throughout the porous polymer 405, such that the porous polymer 405 may include portions that are essentially nonporous as described above with respect to the nonporous polymer 205.

The pores 408 may be distributed uniformly throughout the porous polymer 405, but may also be distributed in different concentrations throughout the porous polymer 405. For example, the porous polymer 405 may have between about 20 and 60 pores per inch ("PPI"). In another example embodiment, the pores 408 may be distributed uniformly throughout the porous polymer 405, but may have different shapes with different dimensions. For example, the pores 408 may be generally circular and have varying diameters throughout the porous polymer 405. The pores 408 may have an average diameter ranging between about 50 µm and 600 µm. The porous polymer 405 may include pores 408 that may have a closed-cell structure or an open-cell structure. In a typical closed-cell structure, each cell may be surrounded by connected faces. An open-cell structure may also have connecting cell faces with a portion of the cell faces open to each other to form passageways or flow channels between them. The passageways are formed by a plurality of interconnected pores 408 which act as flow channels through the porous polymer 405 that may enhance wound therapy including negative pressure wound therapy. The porous polymer 405 may have an open-cell structure formed by the methods known to those skilled in the art including, for example, reticulation methods. In one example embodiment, the porous polymer 405 may be highly reticulated having at least 90% of the pores 408 interconnecting to form the passageways. Fluid flow may be enhanced for a porous polymer 405 with a lower reticulation by utilizing some of the other features described above.

In some embodiments, not according to the invention the porous polymer 405 may be formed from one or more polymer particles. For example, the porous polymer 405 may be a polymer matrix that is formed by fusing polymer particles together. In various embodiments, the porous polymer 405 may include polymer particles that are imperfectly fused to create boundaries between each of the polymer particles that are at least partially intact. By imperfectly fusing the polymer particles together, the pores 408 may be formed between fused portions of the polymer particles. Thus, imperfectly fusing the polymer particles together may provide pores 408 that permit fluid communication of gases and liquids through the polymer matrix, for example, during an application of negative pressure wound therapy. The polymer particles in porous polymer 405 may also be fused by mechanical fastening, adhesive bonding, solvent bonding, co-consolidation, fusion bonding or welding, or any other method known by those having ordinary skill in the art.

The porous polymer 405 is formed using a foaming process. For example, the porous polymer 405 may be formed by mixing polymer particles, such as plastic pellets or powders, with a chemical blowing agent. Subsequently, after the polymer particles are mixed with a blowing agent, the porous polymer 405 may be heated to a high temperature dissolving the chemical blowing agent generating a gaseous reaction product, such as nitrogen or CO₂. The porous polymer 405 may subsequently be subject to a rapid pressure drop to form a foamed porous polymer. In various embodiments, the porous polymer 405 may be formed using at least one of extrusion foaming or injection molded foaming and may include an application of a mechanical foaming agent. One of ordinary skill in the art would know the many type of foaming processes that may be used to form porous polymers. The porous polymer 405 may be formed by a foaming process to increase flexibility of the porous polymer 405 when the porous polymer 405 is in, for example, a sheet form so that a thick polymer sheet may still be sufficiently flexible and to provide apposition forces to a tissue site when the porous polymer 405 is under reduced pressure. In various embodiments, the porous polymer 405 may be subject to suspension polymerization and treated with a polymerization initiator. Once the porous polymer 405 has a polymer chain of a desired length, a terminating agent may be added to stop the reaction.

The porous polymer 405 is a sintered polymer or sintered porous polymer. The porous polymer 405 may be sintered by compacting and forming materials or pieces of a material into a solid mass using at least one of heat or pressure without melting the material to the point of liquefaction. The porous polymer 405 may be sintered to reduce the porosity of the polymer and to thereby enhance at least some properties of the porous polymer 405 including, for example, the strength of the porous polymer 405 while maintaining gas absorbency or fluid flow through the passageways in operation. The porous polymer 405 may be sintered by a firing process where atomic diffusion drives powdered surface elimination in different stages starting from a formation of necks between powders to final elimination of small pores at the end of the firing process. The porous polymer 405 may additionally or alternatively be sintered using methods including plastics sintering, liquid phase sintering, electric current assisted sintering, resistance sintering, spark plasma sintering, electro sinter forging, pressureless sintering, or any other method known by those having ordinary skill in the art.

In some embodiments, the porous polymer 405 may be sintered by a multi-step firing process. For example, at a first stage, the porous polymer 405 may be fired so that atomic diffusion drives powdered surface elimination that forms necks between the powders. At a second stage, the porous polymer 405 may be fired so that atomic diffusion further drives powdered surface elimination for further shaping the powders. At a third stage, the porous polymer 405 may be fired so that atomic diffusion further drives powdered surface elimination to eliminate small pores of the porous polymer 405. In some embodiments, the porous polymer 405 may be sintered by compressing the porous polymer 405 into a solid mass through heat or pressure. The porous polymer 405 may be compressed without melting the porous polymer 405 to the point of liquidation.

In some embodiments, the porous polymer 405 may be formed from beads or particles forming a polymer matrix. The beads or particles forming the polymer matrix may be cleaned and anomalous beads may be filtered out from remaining beads. The beads or particles forming the polymer matrix may subsequently be melted to form a melted polymer. A blowing agent may also be added to the melted polymer. The melted polymer may then be extruded to form the porous polymer 405. In some embodiments, the beads or particles may be pre-expanded using, for example, steam or hot air to reduce a density of the polymer matrix. During pre-expansion, an agitator may be used to keep the bead or particles from fusing together. The pre-expanded beads or particles may then be heated and expanded and subsequently cooled so that the beads or particles harden. The beads or particles may then be fed into a mold of a desired shape to form the polymer matrix. For example, the polymer matrix may be formed into sheets or other forms. At least one of the pores 408 may be formed in the polymer matrix by fusing the beads or particles together using at least one of a heat, a solvent, or a non-solvent. In various embodiments, at least one of the pores 408 may be formed by gaps created when at least two fibers contact, couple, or overlap with each other.

A method of manufacturing a tissue interface such as tissue interface 108 of Figure 1, for a reduced pressure tissue treatment system in accordance with the invention is provided. The method includes foaming a polymer. The polymer may be foamed to form a porous polymer, such as the porous polymer 405 of Figures 4 and 5. The polymer may be foamed by mixing polymer particles, such as plastic pellets or powders, with a chemical blowing agent. The mixture of polymer particles may subsequently be heated to a high temperature dissolving the chemical blowing agent. The blowing agent may be dissolved with the polymer particles to generate a gaseous reaction product, such as nitrogen or CO₂. The mixture of polymer particles may be subject to a rapid pressure drop forming the polymer. It should be understood that while the aforementioned foaming process may be used to form a foamed polymer, the foaming process may additionally or alternatively include extrusion foaming or injection molded foaming, and may include an application of a mechanical foaming agent. One of ordinary skill in the art would know the various types of foaming processes that may be used polymers for a tissue interface 108 as discussed herein. A polymer may be foamed to increase flexibility of the polymer when the polymer is in, for example, a sheet form so that a thick polymer sheet may still be sufficiently flexible while providing apposition forces to a tissue site when the polymer is under reduced pressure.

The methods includes sintering a polymer to form a sintered polymer. The polymer may be sintered by compacting and forming the polymer into a solid mass by at least one of heat or pressure without melting the polymer to the point of liquefaction. The polymer may be sintered to reduce the porosity of the polymer and enhance properties including strength while maintaining gas absorbency. In various embodiments, the polymer may be sintered by a firing process where atomic diffusion drives powdered surface elimination in different stages starting from a formation of necks between powders to final elimination of small pores at the end of the firing process. Polymers may be sintered by plastics sintering, liquid phase sintering, electric current assisted sintering, resistance sintering, spark plasma sintering, electro sinter forging, pressureless sintering, or any other method known by those having ordinary skill in the art.

In various embodiments, a polymer may be sintered by a multi-step firing process. For example, at a first stage, a polymer may be fired so that atomic diffusion drives powdered surface elimination that forms necks between the powders. At a second stage, the polymer may be fired so that atomic diffusion further drives powdered surface elimination for further shaping of the powders. At a third stage, the polymer may be fired so that atomic diffusion further drives powdered surface elimination to eliminate small pores of the polymer. In some embodiments, the polymer may be sintered by compressing the polymer into a solid mass through heat or pressure. The polymer may subsequently be compressed without melting the polymer to the point of liquidation.

In some embodiments, methods of manufacturing a tissue interface such as tissue interface 108 of Figure 1, for a reduced pressure tissue treatment system in accordance with this specification may include expanding a polymer of the tissue interface to form an expanded polymer. For example, a polymer may be expanded from an original size to a size that is between about two and about ten times the original size of the polymer. In some embodiments, the polymer may be expanded before or after the polymer is foamed. In some embodiments, the polymer may be foamed and expanded at the same time. For example, at least some polymers may be expanded by heating the polymer with steam or hot air. The heating may be carried out in a vessel containing the polymer. An agitator may be added to the polymer during heating to keep the polymer from fusing. The expanded polymer may be forced to the top of the vessel separating from unexpanded polymer due to density differences created by the application of heat. Subsequently, the expanded polymer may be cooled to harden the expanded polymer. In some embodiments, the polymer may be expanded before the polymer is sintered. For example, after the polymer is expanded, the polymer may be sintered to form a sintered polymer.

The method of manufacturing a tissue interface such as tissue interface 108 of Figure 1, for a reduced pressure tissue treatment system in accordance with this invention includes texturing a polymer of the tissue interface to form a textured polymer. In some embodiments, the polymer may be textured to form a mesh pattern. The polymer may be textured by embossing or vacuuming forming polymer. In some embodiments, the polymer may be textured to form dimples or channels on a surface of the polymer as discussed herein. When a tissue interface, such as, for example, the tissue interface 108 of Figure 1, includes a polymer that is textured, the tissue interface may provide an apposition force to the tissue site when the tissue interface receives reduced pressure.

The method of manufacturing a tissue interface such as a tissue interface 108 of Figure 1, for a reduced pressure tissue treatment system in accordance with this invention includes sterilizing the tissue interface to form a sterile tissue interface at any stage through a process of manufacturing the tissue interface. For example, a porous polymer may be sterilized before or after the porous polymer is sintered, or before or after the porous polymer is expanded, before or after the porous polymer is textured, or before or after the porous polymer is shaped. Sterilization may include using gamma radiation, electron beam radiation, neutron radiation, ultraviolet light, microwave radiation, heat, supercritical CO₂, ethylene oxide, a chemical biotoxin, or a combination thereof. Chemical biotoxins may include but are not limited to a peracid, a peracid salt, an azide, and ozone. Peracids may include but are not limited to a permangenate salt, hydrogen peroxide, benzoyl peroxide, perbromic acid, periodic acid, perflouric acid, perchloric acid, or a combination thereof.

The methods described herein may provide significant advantages. For example, the tissue interface 108 can reduce trauma and facilitate ease of removal when treating both deep and shallow wounds. The tissue interface 108 encourages granulation without the disadvantage of tissue ingrowth resulting in pain or discomfort upon removal.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

## Claims

1. A method of manufacturing a tissue interface for providing reduced pressure to a tissue site, the method comprising:
foaming a plurality of polymer particles to form a porous polymer;
sintering the porous polymer to form a sintered porous polymer;
texturing the sintered porous polymer to form a textured sintered polymer that is porous; and
sterilizing the tissue interface.

2. The method of Claim 1, wherein texturing the sintered porous polymer comprises at least one of embossing or vacuum forming.

3. The method of Claim 1, wherein foaming the plurality of polymer particles comprises fusing the polymer particles to form a polymer matrix.

4. The method of Claim 3, wherein fusing the polymer particles to form the polymer matrix comprises imperfectly fusing the polymer particles.

5. The method of Claim 3, wherein the polymer particles are fused using at least one of heat, a solvent method, or a non-solvent method.

6. The method of Claim 3, wherein the polymer matrix includes one or more pores configured to provide fluid communication through the polymer matrix.

7. The method of Claim 1, wherein texturing the sintered porous polymer forms one or more channels that include a width between about 0.2 millimeters (mm) and about 1.0 mm, and wherein texturing the sintered porous polymer forms one or more channels that include a depth between about 0.2 mm and about 1.0 mm.

8. The method of Claim 1, further comprising forming at least one of the porous polymer, the sintered porous polymer, or the textured sintered polymer that is porous into one or more sheets.

9. The method of Claim 8, wherein the one or more sheets each comprise a thickness between about 1.0 millimeter (mm) and about 30.0 mm.

10. The method of Claim 8, wherein the one or more sheets are rolled into a roll for dispensing.

11. The method of Claim 1, wherein the polymer particles include a blowing agent configured to be activated by at least one of heat or light to form the polymer particles into the porous polymer.

12. The method of Claim 1, further comprising expanding at least one of the porous polymer, the sintered porous polymer, or the textured sintered polymer that is porous.

13. The method of Claim 12, wherein expanding at least one of the porous polymer, the sintered porous polymer, or the textured sintered polymer that is porous comprises expanding at least one of the porous polymer, the sintered porous polymer, or the textured sintered polymer that is porous from an original size to a size that is between about two and about ten times larger than the original size.

14. The method of Claim 1, wherein the textured sintered polymer that is porous is configured to provide an apposition force to the tissue site when the tissue interface is receiving reduced pressure.

15. The method of claim 1, wherein sterilizing the tissue interface comprises sterilizing the textured sintered polymer that is porous.

## Patentansprüche

1. Ein Verfahren zum Herstellen einer Gewebegrenzfläche zum Bereitstellen von Unterdruck an eine Gewebestelle, wobei das Verfahren umfasst:
Schäumen einer Mehrzahl von Polymerteilchen, um ein poröses Polymer zu bilden;
Sintern des porösen Polymers, um ein gesintertes poröses Polymer zu bilden;
Texturieren des gesinterten porösen Polymers, um ein texturiertes gesintertes Polymer, das porös ist, zu bilden; und
Sterilisieren der Gewebegrenzfläche.

2. Das Verfahren nach Anspruch 1, wobei Texturieren des gesinterten porösen Polymers mindestens eines von Prägen oder Vakuumformen umfasst.

3. Das Verfahren nach Anspruch 1, wobei Schäumen der Mehrzahl von Polymerteilchen das Verschmelzen der Polymerteilchen umfasst, um eine Polymermatrix zu bilden.

4. Das Verfahren nach Anspruch 3, wobei Verschmelzen der Polymerteilchen, um die Polymermatrix zu bilden, unvollkommenes Verschmelzen der Polymerteilchen umfasst.

5. Das Verfahren nach Anspruch 3, wobei die Polymerteilchen unter Verwendung von mindestens einem von Wärme, einem Lösungsmittelverfahren oder einem Nicht-Lösungsmittelverfahren verschmolzen werden.

6. Das Verfahren nach Anspruch 3, wobei die Polymermatrix eine oder mehrere Poren aufweist, die konfiguriert sind, um Fluidverbindung durch die Polymermatrix hindurch bereitzustellen.

7. Das Verfahren nach Anspruch 1, wobei Texturieren des gesinterten porösen Polymers einen oder mehrere Kanäle bildet, die eine Breite zwischen etwa 0,2 Millimeter (mm) und etwa 1,0 mm aufweisen, und wobei Texturieren des gesinterten porösen Polymers einen oder mehrere Kanäle bildet, die eine Tiefe zwischen etwa 0,2 mm und etwa 1,0 mm aufweisen.

8. Das Verfahren nach Anspruch 1, ferner umfassend Bilden mindestens eines des porösen Polymers, des gesinterten porösen Polymers oder des texturierten gesinterten Polymers, das porös ist, in eine oder mehrere Lagen.

9. Das Verfahren nach Anspruch 8, wobei die eine oder mehreren Lagen jeweils eine Dicke zwischen etwa 1,0 Millimeter (mm) und etwa 30,0 mm umfassen.

10. Das Verfahren nach Anspruch 8, wobei die eine oder mehreren Lagen zum Abgeben in eine Rolle gerollt werden.

11. Das Verfahren nach Anspruch 1, wobei die Polymerteilchen ein Treibmittel enthalten, das konfiguriert ist, um durch mindestens eines von Wärme oder Licht aktiviert zu werden, um die Polymerteilchen in das poröse Polymer zu bilden.

12. Das Verfahren nach Anspruch 1, ferner umfassend Expandieren mindestens eines des porösen Polymers, des gesinterten porösen Polymers oder des texturierten gesinterten Polymers, das porös ist.

13. Das Verfahren nach Anspruch 12, wobei Expandieren mindestens eines des porösen Polymers, des gesinterten porösen Polymers oder des texturierten gesinterten Polymers, das porös ist, Expandieren mindestens eines des porösen Polymers, des gesinterten porösen Polymers oder des texturierten gesinterten Polymers, das porös ist, aus einer ursprünglichen Größe auf eine Größe, die zwischen etwa zwei und etwa zehn Mal größer als die ursprüngliche Größe ist, umfasst.

14. Das Verfahren nach Anspruch 1, wobei das texturierte gesinterte Polymer, das porös ist, konfiguriert ist, um der Gewebestelle eine Appositionskraft bereitzustellen, wenn die Gewebegrenzfläche Unterdruck aufnimmt.

15. Das Verfahren nach Anspruch 1, wobei Sterilisieren der Gewebegrenzfläche Sterilisieren des texturierten gesinterten Polymers, das porös ist, umfasst.

## Revendications

1. Procédé de fabrication d'une interface tissulaire pour fournir une pression réduite à un site tissulaire, le procédé comprenant :
le moussage d'une pluralité de particules polymères pour former un polymère poreux ;
le frittage du polymère poreux pour former un polymère poreux fritté ;
la texturisation du polymère poreux fritté pour former un polymère fritté texturé qui est poreux ; et
la stérilisation de l'interface tissulaire.

2. Procédé selon la revendication 1, dans lequel la texturisation du polymère poreux fritté comprend au moins un parmi un gaufrage ou un formage sous vide.

3. Procédé selon la revendication 1, dans lequel le moussage de la pluralité de particules polymères comprend le fusionnement des particules polymères pour former une matrice polymère.

4. Procédé selon la revendication 3, dans lequel le fusionnement des particules polymères pour former la matrice polymère comprend un fusionnement imparfait des particules polymères.

5. Procédé selon la revendication 3, dans lequel les particules polymères sont fusionnées en utilisant au moins un parmi de la chaleur, un procédé au solvant, ou un procédé sans solvant.

6. Procédé selon la revendication 3, dans lequel la matrice polymère inclut un ou plusieurs pores configurés pour fournir une communication fluidique à travers la matrice polymère.

7. Procédé selon la revendication 1, dans lequel la texturisation du polymère poreux fritté forme un ou plusieurs canaux qui incluent une largeur entre environ 0,2 millimètre (mm) et environ 1,0 mm, et dans lequel la texturisation du polymère poreux fritté forme un ou plusieurs canaux qui incluent une profondeur entre environ 0,2 mm et environ 1,0 mm.

8. Procédé selon la revendication 1, comprenant en outre le formage d'au moins un parmi le polymère poreux, le polymère poreux fritté, ou le polymère fritté texturé qui est poreux en une ou plusieurs feuilles.

9. Procédé selon la revendication 8, dans lequel la ou les feuilles comprennent chacune une épaisseur comprise entre environ 1,0 millimètre (mm) et environ 30,0 mm.

10. Procédé selon la revendication 8, dans lequel la ou les feuilles sont enroulées en un rouleau pour distribution.

11. Procédé selon la revendication 1, dans lequel les particules polymères incluent un agent d'expansion configuré pour être activé par au moins une parmi de la chaleur ou de la lumière pour former les particules polymères en le polymère poreux.

12. Procédé selon la revendication 1, comprenant en outre l'expansion d'au moins un parmi le polymère poreux, le polymère poreux fritté, ou le polymère fritté texturé qui est poreux.

13. Procédé selon la revendication 12, dans lequel l'expansion d'au moins un parmi le polymère poreux, le polymère poreux fritté, ou le polymère fritté texturé qui est poreux comprend l'expansion d'au moins un parmi le polymère poreux, le polymère poreux fritté, ou le polymère fritté texturé qui est poreux à partir d'une taille originale jusqu'à une taille qui est comprise entre environ deux et environ dix fois plus grande que la taille originale.

14. Procédé selon la revendication 1, dans lequel le polymère fritté texturé qui est poreux est configuré pour fournir une force d'apposition au site tissulaire lorsque l'interface tissulaire est en train de recevoir la pression réduite.

15. Procédé selon la revendication 1, dans lequel la stérilisation de l'interface tissulaire comprend la stérilisation du polymère fritté texturé qui est poreux.
